# EUROPEAN PATENT APPLICATION

(11) **EP 2 702 998 A1**
(43) Date of publication of application: **05.03.2014**
(21) Application number: 12182576.4
(22) Date of filing: 31.08.2012
(51) Int. Cl.: A61K 31/7105, G01N 33/50, A61P 13/12

(54) **Therapeutic and diagnostic miRNA regulator in kidney disease**

(71) Applicant: IMBA-Institut für Molekulare Biotechnologie GmbH, 1030 Wien (AT)
(72) Inventor: Martinez, Javier, 1030 Vienna (AT); Gebeshuber, Christoph, 4523 Neuzeug (AT)
(74) Representative: Sonn & Partner Patentanwälte

(57) **Abstract**

Summary:
The present invention discloses a method of diagnosing glomerular kidney disease or the risk of glomerular kidney disease comprising determining the expression level of miRNA miR-193a,

wherein said glomerular kidney disease or risk thereof is detected if miR-193a expression levels are increased as compared to a healthy control, method of prophylactically treating a glomerular kidney disease or of reducing the risk of glomerular kidney disease progression in a patient comprising inhibiting miR-193a in a podocyte cell in said patient and suitable means for said methods.

## Description

The present invention relates to glomerulosclerosis regulatory genes, methods of diagnosis and therapy.

Glomerulosclerosis refers to a hardening of the glomerulus in the kidney. It is a general term to describe scarring of the kidneys' tiny blood vessels, the glomeruli, the functional units in the kidney that filter urine from the blood.

Proteinuria (large amounts of protein in urine) is one of the signs of glomerulosclerosis. Scarring disturbs the filtering process of the kidneys and allows protein to leak from the blood into urine.

Focal segmental glomerulosclerosis accounts for approximately 20% of cases of the nephrotic syndrome in children and 40% of such cases in adults. It is the most common primary glomerular disorder causing end-stage renal disease. Its primary feature is progressive glomerular scarring. Early in the disease course, glomerulosclerosis is both focal, involving a minority of glomeruli, and segmental, affecting a portion of the glomerular globe. With progression, more widespread and global glomerulosclerosis develops. The discovery that mutations in podocyte genes are associated with genetic focal segmental glomerulosclerosis has advanced the field of podocyte biology and stimulated new approaches to diagnosis and therapy.

The publication WO 03/082202 A2 describes a method for detecting a kidney glomerular disorder by screening for expression of nephrin gene RNA in a human urine sample.

The EP1336845 A1 discloses the diagnosis of renal disease comprising assaying for urinary sediments with podocytes, tubule epithelial cells, membrane proteins, and podocalyxin and nephrin present in a podocyte.

Reduction in WT1 gives rise to crescentic glomerulonephritis (Morrison et al. Am J Physiol Renal Physiol 295, F12-F17 (2008)), a disease with glomerular crescent-shaped scars, and glomerulosclerosis (Chau et al. PLoS Genet. 7, e1002404 (2011)).

Despite the identification of many factors, including podocyte proteins, circulating podocyte-toxic factors and viral infections, that lead to glomerulosclerosis, approximately 80% of cases are idiopathic.

Glomerulosclerosis is the result of dysregulation of constitutive podocyte proteins that causes collapse of the podocyte shape and of the glomerular filtration barrier. However, the extent to which in idiopathic human glomerulosclerosis these isolated molecular defects are acting alone or in concert and how this could be regulated by a molecular network is unknown.

Therefore it is an objective to provide new diagnostic and therapeutic targets for glomerulosclerosis. Once a causative molecular factor is identified, it can be targeted for therapeutic applications and also assayed in diagnostic methods, in particular screening methods in an early stage of the disease.

These goals are achieved by the present invention. In a first aspect, the invention provides a method of prophylactically treating a glomerular kidney disease or of reducing the risk of glomerular kidney disease in a patient comprising inhibiting miR-193a in a podocyte cell in said patient.

In a related aspect the present invention provides a miR-193a inhibitor for use in treating a glomerular kidney disease or of reducing the risk of glomerular kidney disease in a patient comprising administering said inhibitor to said patient. The invention also relates to the use of a miR-193a inhibitor for the manufacture of a pharmaceutical composition for treating a glomerular kidney disease or of reducing the risk of glomerular kidney disease.

Also provided is a method of reducing or eliminating miR-193a activity in a kidney cell comprising introducing a miR-193a inhibitor into said kidney cell.

The invention provides in a further aspect a method of diagnosing glomerular kidney disease or the risk of glomerular kidney disease comprising determining the expression level of miRNA miR-193a, wherein said glomerular kidney disease or risk thereof is detected if miR-193a expression levels are increased as compared to a healthy control.

In a related aspect, the invention provides a kit with a probe with at least 90% complementary with a miR-193a sequence. Said kit is suitable for performing the diagnostic method. Therefore the present invention also comprises the use of the inventive kit for performing the inventive methods.

The following description equally relates to the inventive methods, inhibitors and kits. The inventive inhibitors and kits can be used in the inventive methods and the description of the inventive methods can be indications for use of the inventive inhibitors. The inventive kit can further be suitable to perform any one of the inventive particular preferred embodiments and may comprise means to this thereto. The present invention is further defined in the claims.

Both the inventive treatment methods and diagnostic methods relate to teaching or diagnosing glomerular kidney disease. A glomerular kidney disease is e.g. glomerulosclerosis or glomerulonephritis. Particular preferred examples of glomerular kidney diseases are associated with abnormal glomerular cell proliferation and/or scaring. Preferably the disease is focal segmental glomerulosclerosis (FSGS). Syndromes in which the WTlgene is disrupted include Frasier syndrome (FS), Denys-Drash syndrome (DDS), congenital nephrotic syndrome, crescentic glomerulonephritis and mesangial sclerosis. These are further preferred examples for glomerular kidney diseases. By increasing WT1 expression via reduced miR-193a inhibition any of these conditions can be ameliorated. In particular, the present invention provides the first individual microRNA that controls the stability of podocytes and causes the pathogenesis of glomerular kidney disease, such as e.g. human focal-segmental glomerulosclerosis if overexpressed.

MicroRNAs (miRNAs) are powerful regulators of gene expression that suppress their target genes by binding to partially or fully complementary sequences and cause inhibition of translation or mRNA degradation, respectively. Ordered miRNA expression is pivotal during organ development and its dysregulation contributes to many diseases. Despite this, no miRNA has so far been identified to contribute to glomerulosclerosis. According to the present invention the expression of miR-193a is greatly increased in glomerulosclerosis and initiates a cascade of molecular events that result in proteinuria, podocyte flattening and loss of foot process architecture. miR-193a (NCBI database accession: NR_029710; miRBase accession: MI0000487, both incorporated herein by reference) primarily acts by suppressing the transcription factor WT1, a master switch of podocyte development, maturation and homeostasis (Morrison et al., supra). Loss of WT1 subsequently drives down-regulation of genes critical for podocyte stability, including podocalyxin and nephrin, and is eventually followed by a catastrophic dysregulation and collapse of the entire podocyte architecture.

In preferred embodiments of the present invention miR-193a comprises one of the sequences selected from SEQ ID NOs: 1, 2, or 3:
Stem loop miR-193a precursor: CGAGGAUGGGAGCUGAGGGCUGGGUCUUUGCGG-GCGAGAUGAGGGUGUCGGAUCAACUGGCCUACAAAGUCCCAGUUCUCGGCCCCCG (SEQ ID NO: 1; MI0000487);
Mature miR-193a (on the 5p precursor arm): UGGGUCUUUGCGGGCGAGAU-GA (SEQ ID NO: 2);
Mature miR-193a complementary strand (on the 3p precursor arm): AACUGGCCUACAAAGUCCCAGU (SEQ ID NO: 3);

SEQ ID NOs 1-3 are human sequences. Of course the invention also contemplates the use of any other homologous miR-193a sequences, including those of any other animal such as mice. The mouse miR-193a-5p sequence is disclosed as ugggucuuugcgggcAagau- ga (SEQ ID NO: 11, miRBase accession: MI0000235, capitalized "A" indicates a difference to the human miR-193a-5p sequence). Homologous mir-193a sequences are published in the miRBase database under accession numbers MI0000235, MI0000487, MI0002029, MI0002030, MI0005014, MI0007564, MI0007655, MI0008159, MI0008566, MI0010034, MI0010171, MI0012773, MI0013051, MI0013113, MI0013813, MI0014855, MI0019413, MI0020721, MI0023355, MIMAT0000459, MIMAT0003794, MIMAT0003795, MIMAT0009635, MIMAT0019157, MI0002031 (all incorporated herein by reference) .

In diagnostic methods, it is preferred to detect at least one of the mature sequences, SEQ ID NO: 2 and/or SEQ ID NO: 3, or the homologous variant in the subject animal under investigation. For therapeutic uses, it is possible to inhibit the precursor (SEQ ID NO: 1) or one or both of the mature miRNAs (SEQ ID NO: 2 and/or SEQ ID NO: 3) or the homologous variant in the subject animal under investigation.

Treating a glomerular kidney disease or of reducing the risk of glomerular kidney disease as in a prophylactic treatment does not require absolute treatment success. A reduction of symptoms of the disease or reducing the risk of further disease progression is within the concept of the present invention. Likewise, diagnosis of such a disease does not necessarily mean that there is a 100% certainty of the disease. The present invention provides at least in formation of a likelihood of the disease in a patient, or that a kidney disease might develop. In particular, miR-193a is an early regulative indicator, that might be upregulated even before the kidney disease has fully developed. Thus miR-193a might be upregulated in a - for the time being - healthy person, who is at risk to develop the disease symptoms. It is particularly preferred to combine the inventive diagnostic and therapeutic methods, which can be performed subsequently if a disease or risk is indicated, e.g. by diagnosing a risk for the kidney disease and prophylactically inhibit miR-193a.

For inhibition of a miR-193a sequence, a miR-193a inhibitor can be used, which inhibits the activity of miR-193a in cells. A miR-193a inhibitor preferably comprises a complementary sequence to the targeted miRNA sequence, binds said miRNA and inactivates it. Conveniently the inhibitor is a single or double stranded nucleic acid molecule. Preferably, the inhibitor of miR-193a is a single-stranded molecule comprising a sequence which is fully or partially complementary to the miR-193a guide strand sequence or the miR-193a-5p sequence, e.g. to the sequence set out in SEQ ID NO: 2 or 11, or of the passenger strand or the miR-193-3p sequence, e.g. to the sequence set out in SEQ ID NO: 3, or to a homologous sequence such as SEQ ID NO: 11. These inhibitors function in a manner similar to antisense RNA, i.e. by binding to the target miR-193a sequence and preventing it from being incorporated into RISC. The miR-193a inhibitor can be RNA or DNA. DNA is preferred. RNA can be a preferred embodiment if one or more further stabilizing modifications are included.

MiRNA inhibitors may comprise nucleotide modifications to improve stability and/or inhibition activity as e.g. disclosed in the publication WO 2006/137941 A2 (incorporated herein by reference), such as a biotin, an amine group, a lower alkylamine group, an acetyl group, 2'O-Me (2'oxygen-methyl), DMTO (4,4'-dimethoxytrityl with oxygen), fluoroscein, a thiol, or acridine as replacement of an OH or phosphate on the 5' nucleotide or on the first or last 2 to 4 sugar residues. Further preferred modifications are modifications to increase ribonuclease resistance, such as modifications of the nucleic acid backbone, in particular LNA modifications. In an LNA nucleotide the 2' oxygen and 4' carbon are connected. Preferably the inventive inhibitor comprises LNA, which is particularly preferred as it also increases hybridization properties and thus inhibition activity. In further embodiments or in combination, the inhibitor comprises a thiophosphate backbone.

In particular according to a preferred embodiment of the present invention said inhibitor comprises a nucleic acid modification which increases nuclease resistance or increases binding affinity with a miR-193a sequence as determined by a decrease in the melting temperature of the hybridized complex of the miR-193a inhibitor with the miR-193a sequence as compared to a miR-193a inhibitor without said modification.

An miRNA inhibitor is preferably between about 14 to 30 nucleotides in length and comprises a 5' to 3' sequence that is at least 90% complementary to the 5' to 3' sequence of a mature miRNA. In certain embodiments, an miRNA inhibitor molecule is 14, 15, 16, 17, 18, 19, 20, 21, 22, 23, 24, 25, 26, 27, 28, 29 or 30 nucleotides in length, or any range derivable therein. Moreover, an miRNA inhibitor has a sequence (from 5' to 3') that is or is at least 90%, 91%, 92%, 93%, 94%, 95%, 96%, 97%, 98%, 99% or 100% complementary, or any range derivable therein, to the 5' to 3' sequence of a mature miRNA. Preferably said miR-193a sequence comprises at least 16 consecutives nucleotides of any sequence as set forth in SEQ ID NO: 1, SEQ ID NO: 2 or SEQ ID NO:3 or a homologous variant, such as SEQ ID NO: 11. The nucleic acid inhibitor can be 15 to 30 nucleotides in length.

According to the invention, the miR-193a inhibition is specific to miR-193a, e.g. not a global ablation of miRNAs by suppression of the miRNA processing enzymes Dicer or Drosha.

Alternatively, or in addition to administering an inhibitory nucleotide as described above it is also possible to administer a vector encoding said nucleic acid, wherein said vector causes expression of said nucleic acid inhibitor in a cell. Both the inhibitory nucleotide as well as the vector are collectively referred to as miR-193a inhibitors herein. The vector causes expression of an inhibitory nucleotide in a cell, in particular in a kidney cell, more preferred in a podocyte cell, therein consequently the inhibitory nucleotide binds and inactivates endogenous miR-193a in said cell. Suitable expression vectors, with known kidney specific promoter regions as known in the art can be used.

The inhibitor is usually administered in a therapeutically effective amount, an amount that reduces miR-193a activity in a cell. In particular embodiments the miR-193a activity may be suppressed to normal levels of an average person, furthermore, the miR-193a activity may be reduced below average levels. Preferably the miR-193a activity is reduced by at least 25%, by at least 30%, by at least 40%, by at least 50%, by at least 60%, by at least 70% by at least 80% or by at least 90% as compared without inhibition. In preferred embodiments this reduction equates to miR-193a concentration.

Inhibitor of the invention may be administered by injection, either subcutaneous, intravenous or intramuscular, or by oral, nasal, pulmonary or rectal administration. The route of administration eventually chosen will depend upon a number of factors and may be ascertained by one skilled in the art.

Also encompassed are compositions comprising miR-193a inhibitors modified with water soluble polymers to increase solubility, stability, plasma half-life and bioavailability. Compositions may also comprise incorporation of miR-193a inhibitors into liposomes, microemulsions, micelles or vesicles for controlled delivery over an extended period of time. MiR-193a inhibitors may be formulated into microparticles suitable for pulmonary administration. Such formulations are preferably used or are suitable for intracellular delivery, preferably into kidney cells.

The invention also provides for pharmaceutical compositions comprising a therapeutically effective amount of the miR-193a inhibitor to cells and tissues as part of a miR-inhibitory or anti-sense therapy regimen.

Any of the above can be combined with WT1 expression therapy, wherein WT1 expression is increased in kidney cells as described above. WT1 expression therapy preferably comprises administering a WT1 gene or vector comprising said gene into kidney cells in a patient. Similar formulations and routes for administration as described for miR-193a inhibitors can be used.

The present invention also provides a method of reducing or eliminating miR-193a activity in a kidney cell comprising introducing a miR-193a inhibitor into said kidney cell. This method can be an aspect of the present inventive therapeutic treatment or use of an inhibitor or it can be an in vitro method.

In the inventive treatment method or in vitro method preferably said kidney cell is a visceral epithelial kidney cell, preferably a cell of the Bowman's capsule of the kidney, in particular preferred a podocyte cell. In addition, or alternatively, the cell is a cell with overexpression of miR-193a as compared to a healthy cell of the same kind, e.g. a podocyte cell. A cell with overexpression of miR-193a preferably comprises miR-193a concentration that is at least 1.5x, 2x, 3x or 4x, or more or any interval in between these amounts, as compared to the healthy cell. The cell might be a cell with insufficient WT1 expression. Insufficient WT1 expression might be less than 2/3, less than 1/2, less than 1/3, less than 1/4 less than 1/6 of the expressed concentration of WT1 in a healthy cell.

Preferred embodiments of the inventive methods comprise the step of comparing one or more characteristics of a kidney cell having the miR-193a inhibitor with a kidney cell not having the miR-193a inhibitor and/or assaying the cell for expression of mature miR-193a. In certain embodiments, the cells with the miR-193a inhibitor may be compared to cells in which a different molecule was introduced (such as a negative control that does not include an miR-193a or miR-193a inhibitor or a different miRNA). It is contemplated that the compared cells need not be evaluated at the same time. In fact, the comparison cells need not have been cultured at the same time; one may refer to a report or previous observation.

Other methods include reducing or eliminating activity of one or more miR-193a from a cell comprising introducing into a cell an miR-193a inhibitor. In certain embodiments, methods also include comparing one or more characteristics of a cell having the miR-193a inhibitor with a cell not having the miR-193a inhibitor.

The inventive method of detecting miR-193a can be used to provide information on a diseased state or a risk for a disease, in particular wherein the disease is a kidney disease as described above. Therefore the present invention also provides a method of diagnosing glomerular kidney disease or the risk of glomerular kidney disease comprising determining the expression level of miR-193a, wherein said glomerular kidney disease or risk thereof is detected if miR-193a expression levels are increased as compared to a healthy control. The control samples of a healthy or known diseased reference might be of the same type as the sample from the patient.

Said expression can be determined in a cell, especially a kidney cell as mentioned above. A more convenient way is to determine urine samples. miR-193a might be detected in the urine fluid or in the urine sediment, in cells or in urinary exosomes. Such a sediment, cells or exosomes are preferably enriched in the inventive methods. Cells and exosomes can be enriched by antibody binding or other immunoaffinity purification methods. Another enrichment method for sediment, cells and even exosomes comprises centrifugation and obtaining the sediment, cell or exosome fraction.

The present invention further relates to a kit suitable for performing a diagnostic or detection or comparison method, comprising a probe with at least 90% complementary with a miR-193a sequence, wherein said miR-193a sequence comprises at least 16 consecutives nucleotides of any miR-193a sequence, in particular a sequence as set forth in SEQ ID NO: 1, SEQ ID NO: 2 or SEQ ID NO: 3 or any homologous miR-193a variant thereof such as mouse miR-193a sequences including SEQ ID NO: 11, a sample holding means, preferably a centrifugation vial, and one or more comparative sample holding means with a comparative miR-193a amount of a healthy control and/or of a kidney diseased reference. Such kidney diseases are mentioned above, e.g. FSGS.

The kit may further comprise one or more standards with given concentrations of miR-193a for signal comparison.

Preferably the kit also comprises a labelled nucleotide, which binds to the probes or alternatively to the miR-193a to be detected. The labelled nucleotides binding the probes can be used to detect the absence of miR-193a binding in a competitive assay. miR-193a presence prevents binding to the probes and reduces the signal in concentration dependence.

Preferably the probes are immobilized such as on a solid support, like a microarray. The solid support preferably also comprises in isolated spots a positive and/or negative control for signal normalization. Such controls are independent on miR-193a state in a sample.

The present invention is further illustrated by the following figures and examples, without being limited to these particular embodiments of the invention.

### Figures

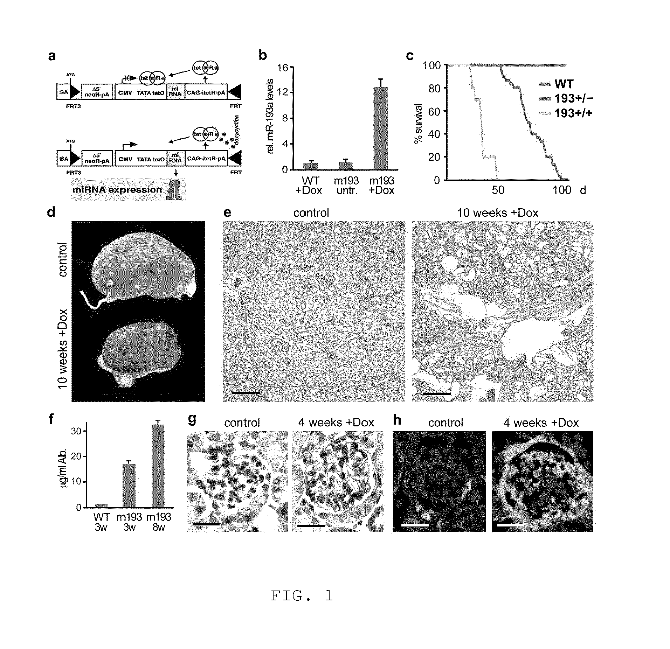

***Figure 1******.** Doxycycline-induced miR-193a transgenic mice develop a renal phenotype.*
   **(a)** Scheme of the doxycycline (Dox)-inducible miR-193a construct. **(b)** Expression levels of miR-193a in wild-type (WT) and transgenic (m193) mice without (untr.) and with (+Dox) induction of miR-193a expression by Dox. **(c)** Kaplan-Meier survival analysis of wild type (WT), heterozygous (193+/-) and homozygous (193+/+) miR-193a over-expressing mice. **(d)** Macroscopic appearance of the kidneys of control mice and after 10 weeks of miR-193a induction by Dox. **(e)** Survey views of kidney sections of control mice and after 10 weeks of induction of miR-193a by Dox (hematoxylin and eosin stain).
   **(f)** Progressive albuminuria after miR-193a-induction as determined by ELISA. The y-axis indicates µg albumin per ml urine.
   **(g)** Preferential localization of miR-193a by *in situ* hybridization in glomerular podocytes at four weeks after miR-193a induction, as visualized by digoxigenin *in situ* hybridization. Controls fail to hybridize under the same conditions.
   **(h)** In controls localization of miR-193a by fluorescent *in situ* hybridization reveals expression in some capsular epithelial cells and of traces in podocytes. Massive expression is obtained in podocytes and capsular epithelial cells at 4 weeks of miR-193a induction. Scale bars: 100 µm **(e),** 10 µm **(g, h).**

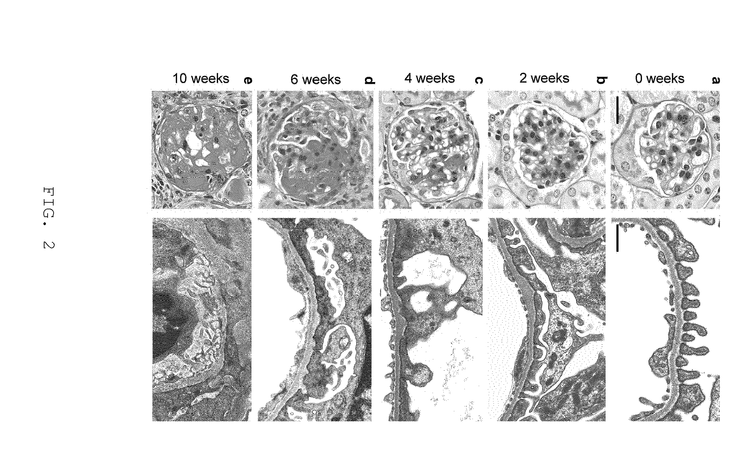

***Figure* 2****.** *Development of glomerular lesions induced by over-expression of miR-193a after two, four, six and ten weeks.* **(left panel)** The glomerular pathology develops from a normal structure into a typical FSGS of increasing severity and results in total glomerular sclerosis after 10 weeks (PAS stain). **(right panel)** Representative electron micrographs correspond to the adjacent light micrographs. The morphology of foot processes progresses from normal to complete flattening, depending on the duration of miR-193a induction. Scale bars: 10 µm **(left panel),** 0.1 µm **(right panel).**

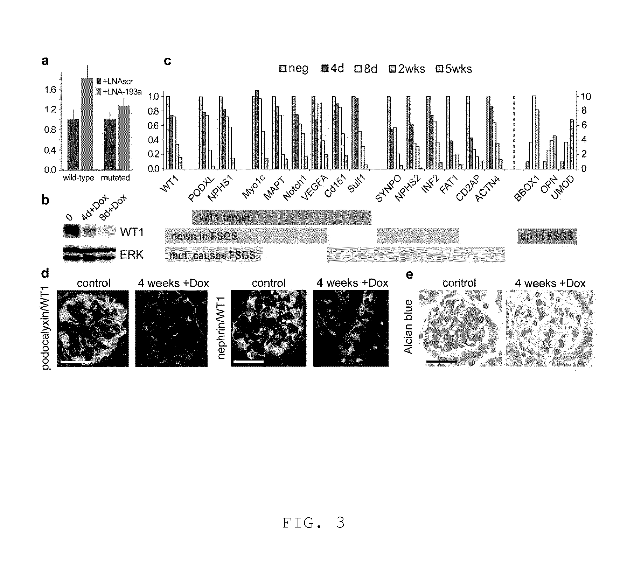

***Figure* 3****.** *miR-193a target genes in podocytes.*
   **(a)** Luciferase reporter gene assay for repression of WT1 by miR-193a. miR-193a over-expressing cultured mouse podocytes were transiently transfected with wild type WT1 (left columns) or WT1 that was mutated in the putative miR-193a binding site (right columns) plus scrambled LNA or LNA-193a, respectively. The y-axis indicates relative light units (RLUs). **(b)** miR-193a down-regulates WT1 at the protein level as determined by Western blot analysis in extracts of glomeruli from wild type and transgenic mice induced for 4 or 8 days, Erk served as normalization control.
   **(c)** Expression changes of glomerular transcripts after 4 and 8 days, and 2 and 5 weeks after induction of miR-193a, as determined by qPCR. The y-axis indicates relative expression levels of the indicated genes **(d)** Double immunofluorescence labeling for podocalyxin (green) and WT1 (red), or for nephrin (green) and WT1 (red), four weeks after induction of miR-193a. **(e)** Alcian blue staining indicates that the overall electro-negative charge of the glomerulus is highly reduced 4 weeks after miR-193a induction, as compared to controls. Scale bars: 10 µm **(d, e)**

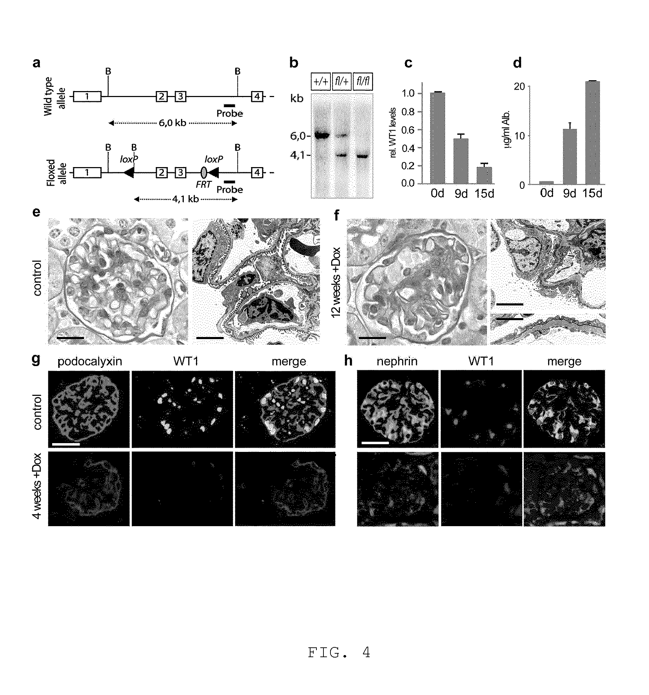

***Figure 4******.** Conditional knock-out of WT1 induces an FSGS phenotype similar* to *overexpression of miR-193a.* **(a)** Scheme of the inducible WT1 knock-out construct. B, *BamHI* restriction site; frt, flip recombinase recognition site; *loxP,* Cre recombinase recognition site. **(b)** Southern blot analysis of *BamHI* restricted tail genomic DNA probed with the 3' probe indicates the presence of a 6.0-kb fragment when DNA from wild-type animals is used as template, and a 4.1-kb fragment in the case of the *Wt1flox* allele in *Wt1f1*/*+* and *Wt1f1*/*f1* mice. **(c)** qPCR analysis in isolated glomeruli shows loss of WT1 mRNA by Dox feeding, at the time periods indicated. **(d)** Development of albuminuria after 9 and 15 days of Dox feeding. **(e)** Feeding of control mice with Dox fails to induce glomerular pathology, as assesed by PAS staining and electron microscopy. **(f)** Development of FSGS with flattening of foot processes at 12 weeks after Dox feeding in *WT1* knock-out mice (PAS stain). **(g)** Double immunofluorescence labeling for podocalyxin (red) and WT1 (green) after 12 weeks of Dox feeding. **(h)** Double immunofluorescence labeling for nephrin (green) and WT1 (red) after 12 weeks of Dox feeding. Scale bars: 10 µm **(d, f),** 1 µm **(electron micrographs),** 15 µm **(g, h)**

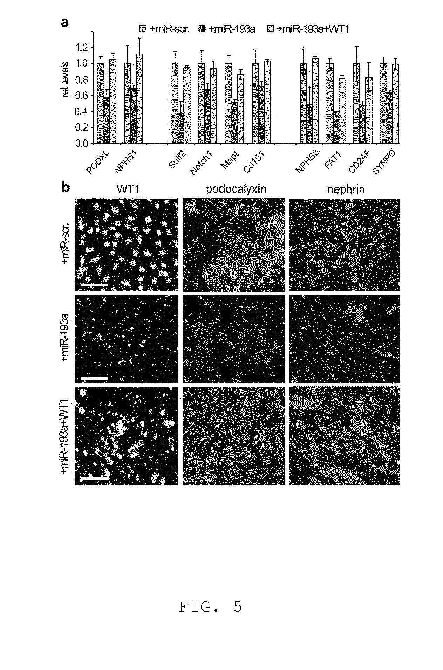

***Figure 5*****.** *miR-193a-induced effects are rescued by WT1 over-expression.*
   **(a)** qPCR analysis of the indicated genes in cultured mouse podocytes that were transiently transfected with scrambled miRNA, miR-193a, or miR-193a plus WT1, respectively. **(b)** Immunofluorescence indicates the expression levels of WT1 (green), nephrin and podocalyxin (red) in cultured mouse podocytes over-expressing scrambled miRNA (upper panels), miR-193a (middle panels), or miR-193a plus WT1 (lower panels). Scale bars: 7.5 µm **(b)**

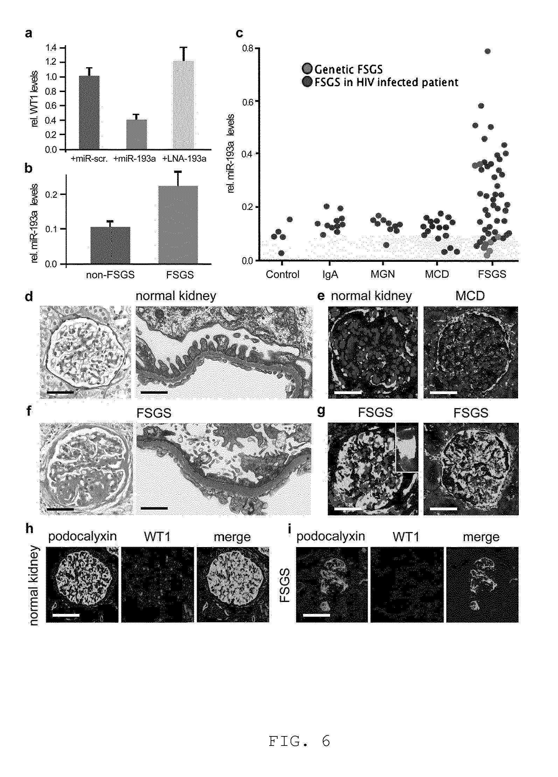

***Figure 6*****.** *miR-193a in cultured human podocytes and in human glomerular diseases.*
   **(a)** Expression of WT1 in cultured human podocytes is reduced by transient transfection with miR-193a, while transfection with LNA-193a fails to significantly enhance WT1 levels. The y-axis indicates relative levels of expression **(b)** Cumulative comparison of mean miR-193a expression in laser capture microdissected glomeruli of patients with FSGS (n=49) *versus* other diseases and the control cases (n=42) reveals over-expression of miR-193a in FSGS (p=0.00004, Mann-Whitney-U test). The y-axis indicates relative expression levels of miR-193a, as determined by qPCR. **(c)** Expression levels of miR-193a in normal controls (n=5), IgA nephropathy (IgA, n=10), membranous nephropathy (MGN, n=10), minimal change disease (MCD, n=17), and FSGS (n=49). FSGS related to a genetic cause (n=4) are indicated by red dots, and FSGS associated with HIV (n=4) by blue dots. The y-axis indicates relative expression levels of miR-193a, as determined by qPCR.
   **(d)** Example of a control case with normal glomerular structure (PAS-stain) and regular foot processes. **(e)** Fluorescent *in situ* hybridization for miR-193a in glomeruli of a normal control and an MCD patient. **(f)** Example of FSGS with typical focal sclerosis (PAS stain) and complete flattening of foot processes. **(g)** Fluorescent *in situ* hybridization of miR-193a in glomeruli of two FSGS patients showing greatly enhanced signals in podocytes over controls. **(h, i)** Double immunofluorescence localization of podocalyxin (green) and WT1 (red) shows high expression in normal human kidneys **(h),** and its reduction in FSGS **(i).** Scale bars: 15 µm **(d-i),** 0.1 µm **(electron micrographs)**

### Examples

An inducible transgenic mouse in which over-expression of miR-193a in podocytes causes glomerular disease was developed, and the underlying pathogenic mechanisms were identified. It is shown that miR-193a suppressed WT1, a master regulator of podocyte development and homeostasis. As consequence, expression of genes that are controlled by the transcription factor WT1 (including nephrin and podocalyxin) was drastically reduced, and this was followed by generalized dysregulation of gene expression and collapse of the podocytes. All data are backed up by adequate rescue experiments. These findings were extended to human FSGS, as it was shown that in microdissected glomeruli of human FSGS patients miR-193a is strongly overexpressed over normal controls and other glomerular diseases. The first and dominant regulatory micro-RNA of podocytes that causes glomerular disease was identified, which was mapped to a novel pathogenic mechanism for glomerulosclerosis.

### Example 1: Transgenic mice overexpressing miR-193a.

Transgenic mice were generated by Taconic-Artemis. The short-hairpin encoding miR-193a was cloned downstream of a Dox-inducible CMV promoter. The modified ROSA26 allele carries the miR-193a coding region under the control of the CMV-tet promoter (CMV TATA teto), the codon optimized tetRi gene under the control of the CAGGS promoter (CAG-itetR-pA), and a truncated neoR gene (neoR-pA) for positive selection of clones upon successful recombinase-mediated cassette exchange (RMCE) via F3/FRT sites as described37. The construct was introduced into the ROSA26 locus of 129Sv/Black6 ES cells by RMCE. ES cells were injected into diploid blastocysts and chimeric mice were generated and backcrossed. For genotyping animals carrying the germline transmitted construct, primers Artemis1958_5 (CGATCAGGATGATCTGGACG (SEQ ID NO: 4)) + Artemis1958_6 (CAAGCTCTTCAGCAATATCAC (SEQ ID NO: 5)) as positive control and 1114_1 (CTCTTCCCTCGTGATCTG-CAACTCC (SEQ ID NO: 6)) + 1114_2 (CATGTCTTTAATCTACCTCGATGG (SEQ ID NO: 7)) as wild-type control were used. For induction (dere-pression) of miR-193a mice were fed with 1 mg/ml Doxycycline in a 5% sucrose solution causing release of tetR. Over-expression of miR-193a was verified by qPCR and Northern blotting, levels of miR-193b appeared unaffected.

### Example 2: Conditional Wt1 knock-out mice.

*LoxP* sites flanking *Wt1* exons 2 and 3 were introduced into the mouse germline by homologous recombination using a targeting vector that encompasses 8.9 kbp of the *Wt1* locus and a *FRT* site flanked neomycin cassette for selection. The latter was eventually removed by crossing of heterozygous *Wt1flox* mice to a Flp deleter line. Cre mediated deletion of *Wt1* exons 2 and 3 should result in a frame shift producing a pre-mature stop codon in exon 4. Presumably, respective mRNA molecules are either subject to nonsense mediated decay (NMD) or translated into truncated, non-functional protein. The inducible podocyte-specific *Wt1* knock-out mouse line was generated by crossing of *Wt1flox* mice with Pod-rtTA and LC-121 mice to obtain *Wt1fl*/*fl;Pod-rtTA;LC-1.* To inactivate *Wt1,* mice were fed with 2 mg/ml Doxycycline in a 5% sucrose solution.

### Example 3: Cell culture.

Immortalized murine and human podocytes were a kind gift of P. Mundel and were handled as described earlier38. Cells stably over-expressing scrambled miRNA, or miR-193a in a pMSCV-puro vector or WT1 in a pMSCV-zeo vector were generated by retroviral gene transfer as described earlier using the Phoenix packaging cell line.

### Example 4: Isolation of RNA and RNA-Seq.

Isolation of RNA from cultured cells, murine and human samples was performed with the RNeasy or the miRNeasy FFPE kits from Qiagen.

Total RNA was subjected to RNA-Seq following the mRNA sequencing protocol provided by Illumina (TruSeq™ RNA Sample Preparation Kit). The first step in the workflow involved purifying the poly-A-containing mRNA molecules using 2 rounds of poly-T-oligo-attached magnetic beads. Following purification, the mRNA were fragmented into small pieces using divalent cations under elevated temperature. The cleaved RNA fragments were copied into first strand cDNA using reverse transcriptase and random primers. This was followed by second strand cDNA synthesis using DNA Polymerase I and RNase H. These cDNA fragments then went through an end repair process, the addition of a single 'A' base, and then ligation of the adapters. The products were then purified and enriched with 15 cycles of PCR (with a size distribution around 200-250bp) to create the final cDNA library. Finally the adapter-ligated DNA was sequenced for 36 cycles on a HiSeq2000 sequencer, according to the manufacturer's instructions. Sequencing reads were processed using RUM, which is an alignment, junction calling, and feature quantification pipeline specifically designed for Illumina RNA-Seq data. Briefly, RUM maps reads in three phases. First it maps against the genome using Bowtie, then it maps against a transcriptome database using Bowtie, then it maps against the genome using Blat. The information from the three mappings was merged into one mapping. This leveraged the advantages of both genome and transcriptome mapping as well as combining the speed of Bowtie with the sensitivity and flexibility of Blat. Only uniquely mapped reads were retained. A feature quantification file was generated that assigned quantified values to genes, exons, introns and junctions using the RPKM (reads per kilobase of exon model per million mapped reads) measure.

### Example 5: miRNA profiling.

RNA was amplified using the MessageAmp II aRNA Amplification Kit (Ambion, #AM1751). Amino-allyl-cDNA was labeled with Alexa 555 or 647, respectively. 4µg cDNA was hybridised on arrays spotted with murine RIKEN sequences. Experiments were performed using two biological replicates. The TaqMan miRNA Reverse Transcription Kit and the 2x TaqMan Gene Expression Kit from Applied Biosystems were used to assess the levels of miR-193a, snoRNA-135 (normalisation for murine samples), and RNU24 (normalisation for human samples).

### Example 6: qPCR.

qPCR for mRNA was performed on a Biorad CFX96 Real Time System with a C1000 Thermal Cycler using the 2xSYBR Green Master-mix from Peqlab. qPCR primers for B2M, cyclophilin A (used for normalization), WT1, podocalyxin, NPHS1, NPHS2, synaptopodin, CD2AP, ACTN4, FAT1, INF2, Myolc, Notch1, Parva, Sulf1, Sulf2, VEGFA, BBOX1, uromodulin, and osteopontin were from Qiagen or Applied Biosystems.

### Example 7: Isolation of murine glomeruli and Western blot analysis.

Isolation of murine glomeruli was performed by the magnetic bead method. The primary antibodies for immunoblotting were monoclonal mouse anti-WT1 (Santa Cruz) and anti-Erk (Sigma).

### Example 8: Site-directed mutagenesis.

The QuickChange mutagenesis kit from Stratagene was used. The respective primers were 5'-CCGACCATCTGAAAACGCACACCAGGAC-3' (SEQ ID NO: 8) and 5'-GTCCTGGTGTGCGTTTTCAGATGGTCGG-3' (SEQ ID NO: 9) for the putative miR-193a seed-binding site (AAGACCCA (SEQ ID NO: 10)) at nt 1398 of the murine WT-1-cDNA.

### Example 9: Reporter gene assays.

5x10⁴ murine podocytes over-expressing miR-193a per well were seeded in a 24-well plate, the experiment was performed in quadruplicates. 200ng of pGL3 vector containing the indicated sequences coupled to firefly luciferase were transfected per well. 100 nM LNA-193a or scrambled LNA were co-transfected per well, respectively. Renilla luciferase was used for normalisation.

### Example 10: Patient samples.

Paraffin blocks of human renal biopsies were obtained from the Biobank/Bioarchive at the Institute of Clinical Pathology at the Medical University Vienna. The use of human samples was permitted by the Ethical Committee of the Medical University of Vienna (permit # 1171/2011), and was carried out in compliance with Austrian legislation. The 91 selected patients included 49 cases with FSGS (including 4 genetic and 4 FSGS with HIV), 17 with MCD, 10 with membranous glomerulonephritis, 10 with IgA Nephropathy and 5 healthy controls. The diagnoses of all cases were confirmed by light and electron microscopy.

### Example 11: Laser capture microdissection.

Formalin-fixed, paraffine embedded patient and mouse samples were cut at 5µm and mounted onto Leica PEN-membrane slides (Leica Microsystems, Wetzlar, Germany). They were deparaffinized, re-dehydrated and lightly stained with haemalaun. Slides were quickly air-dried and subjected to laser microdissection with a Leica LMD6000 Microscope (Leica Microsystems, Wetzlar, Germany). RNA of isolated glomeruli was extracted with a Qiagen miRNeasy FFPE Kit. miR-193a was quantitated by qPCR and normalized to RNU24.

### Example 12: Immunoflourescence.

1x10³ Cells were grown on 8 well chamber slides coated with rat tail collagen I. After differentiation, cells were washed with HBSS with Mg²⁺ and Ca²⁺ (HBSS +/+) (Gibco®, distributed by Invitrogen, Lofer, Austria) and fixed with 4% paraformaldehyde (PFA) for 10 minutes at room temperature (RT). Cells were washed 3 times with phosphate buffered saline (PBS) and permeabilised with 0.1% Triton X-100. After another washing step with PBS, cells were incubated with mouse block for 30 min at RT. After washing with PBS, quenching was performed using 10% goat serum in PBS, followed by incubation with the following primary antibodies: goat anti- Podocalyxin (R&D, AF1556), rabbit anti-CD2AP (SantaCruz, sc-9137) rabbit anti-WT1 (SantaCruz, sc-192), mouse anti-WT1 (Dako, M3561) and mouse anti-synaptopodin (Maine Biotechnology, MAB60105), mouse anti-Vimentin (Zymed 18-0052) Cells were incubated with primary antibody for 1h at 20°C, washed three times with PBS and stained with fluorescent secondary anti-rabbitFc-IgG, anti-mouseFc-IgG and anti-goatFc-gG antibodies (Molecular Probes, goat anti rabbit A-11012, goat anti mouse A-11020, donkey anti goat A-11058) and DAPI (SERVA, Cat#18860).

### Example 13: In situ hybridization on paraffin sections.

We followed a protocol established previously (Michael et al. Lab Invest 23, 649-657, 1970). In short, 5µm thick paraffin sections were prepared RNAse-free. Paraffin was removed by treatment with Xylol (3x 10 min), Ethanol (100%, 75%, 50%, 25%) and DEPC-treated water, then washed 2x for 5 min with PBS. Consecutive blocks with Avidin and Biotin for 10 min were performed, followed by 2 PBS washes for 5 min. Cells were treated with Proteinase K at 37°C for 20 min, followed by a 30 sec glycine in PBS wash and 3 PBS washes for 5 min. Postfixation was performed in 4% formaldehyde for 10 min, followed by 2 PBS washes for 5 min. Sections were acetylated, followed by 2 PBS washes for 5 min. Prehybridization in hybridization solution was performed for 2h, followed by hybridization at 55°C with modified LNA oligos. Washing was performed in 2xSSC for 15 min and 3 washes in 50% formamide in 2xSSC for 30 min at hybridization temperature. Blocking was done for 60 min in 0.5% blocking reagent in TNB buffer at room temperature. Incubation with Strep-tavidin-HRP in TNB buffer was performed for 30 min, followed by 3 washes in PBST for 5 min. TSA Plus Fluorescence Amplification solution was used to enhance the signals.

### Example 14: Alcian blue staining.

De-paraffinized tissue sections were stained in 1% Alcian blue (Sigma) solution and counter-stained with 0.1% fast red41 (Sigma).

### Example 15: Statistical analysis.

Statistical analysis was carried out with SPSS19 (IBM; NY, USA). As normality tests revealed non-Gaussian distribution in the FSGS group, Mann-Whitney U and Kruskal Wallis test for multiple comparison was used for scaled variables as well as Fisher's exact test when appropriate.

### Example 16: Expression of miR-193a in the kidney.

A mouse was generated carrying a miR-193a transgene ubiquitously inducible by doxycycline under the control of a CMV-tet promotor (**Fig**. **1a****, b**). miR-193a was induced in two months old mice and caused death after ∼12 weeks in heterozygotes and in ∼6 weeks in homozygotes (**Fig. 1c****).** Dead mice showed contracted kidneys with tubular atrophy, scarring, cyst formation and focal tubular regeneration (**Fig. 1d****, e**), while other organs were unaffected. Progression of glomerular lesions was accompanied by increasing albuminuria (**Fig. 1f****).** Strong miR-193a signals were obtained by *in situ* hybridization in podocytes, in parietal epithelial cells of Bowman's capsule and distal tubules, but not in interstitial blood vessels or proximal tubules (**Fig. 1g****, h**). Scarce expression of miR-193a was found in podocytes of controls and in capsular epithelial cells of wild type mice (**Fig. 1g****, h**).

### Example 17: miR-193a over-expression in mice causes FSGS.

Minimal irregularities of the glomerular architecture were observed two weeks after doxycycline-induced expression of miR-193a and these progressed to focal sclerosis after 4 weeks, as seen by light microscopy (**Fig. 2a****-c**). Focal sclerosis increased with time, and after 10 weeks the glomeruli were globally sclerotic and showed broad capsular adhesions (**Fig. 2d****, e**). By electron microscopy the foot processes were plump, formed direct contacts, and the slit diaphragms were rarified after two weeks of induction (**Fig. 2b**). From 4 weeks on, podocytes were completely flattened and slit diaphragms were largely replaced by cell junctions in most glomerular loops (**Fig. 2c****-e**). Mouse kidneys that expressed control shRNAs showed no changes in glomerular structure and function.

### Example 18: WT1 is a major target of miR-193a.

Downstream targets of miR-193a were determined in a time course gene expression analysis by RNA-Seq of mRNA extracted from glomeruli isolated with magnetic beads. We have compared the down-regulated genes of the RNA-Seq analysis with predicted miR-193a targets within the genes lost in the human disease. Of the 553 down-regulated human genes, 92 (17%) were putative targets of the miR-193 family. According to the target prediction algorithm miRanda this enrichment was highly specific (p<0.001), suggesting up-regulation and regulatory impact of miR-193a in human FSGS. Only six of these 92 genes had previously been related to podocytes (*ATOH8, COL4A3, FOXC1, GJA-1, Nebl, WT1*) and of these only WT1 had previously been linked to FSGS (Chau at al., supra). WT1 is a master regulator of podocyte homeostasis and so its role was analyzed in miR-193a over-expressing podocytes. A predicted binding site for miR-193a lies at the end of the coding sequence of the *WT1* gene which is identical in mouse and man. Accordingly, a reporter gene assay was performed to determine whether WT1 is a direct target of miR-193a. miR-193a decreased the expression of luciferase coupled to the wild type WT1 sequence, which was decompressed by inhibition of miR-193a by complementary LNA (**Fig.3a**). Western blot analysis confirmed down-regulation of WT1 protein in isolated glomeruli of miR-193a transgenic mice (**Fig 3b**). **It was** focused on a panel of 17 genes that are expressed in podocytes and dysregulated in human FSGS and/or targets of WT1 for further analysis. For these genes expression changes by RNA-Seq and by qPCR (**Fig. 3c**) were confirmed. Among the down-regulated genes were the WT1 targets podocalyxin, nephrin, Notch1, VEGFA and the slit diaphragm components podocin, CD2AP and INF215-18 **(****Fig. 3c****).** In line with reports on human FSGS, levels of gamma-butyrobetaine hydroxylase (BBOX1), osteopontin and uromodulin were strongly up-regulated as reported previously **(****Fig. 3c****).** Taken together, these results identify WT1 as direct miR-193a target and demonstrate its down-regulation in glomeruli of transgenic miR-193a mice.

### Example 19: Podocalyxin and nephrin are effector targets downstream of WT1.

*It was* focused on two prototypic gene products downstream of WT1 that have previously been shown to be essential for podocyte stability, i.e. the sialoglycoprotein podocalyxin (encoded by the *PODXL* gene), and the major slit diaphragm protein nephrin (encoded by the *NPHS1* gene). Loss of WT1, podocalyxin and nephrin was observed by immunofluorescence in glomeruli of transgenic mice at four weeks after induction of miR-193a **(****Fig. 3d****).** Also the labeling of the "glomerular polyanion", an indicator of overall glomerular negative charge, by Alcian blue was strongly reduced at this time point **(****Fig. 3e****).**

### Example 20: Inducible deletion of WT1 phenocopies the miR-193a transgenic mouse.

Constitutive *WT1* knock-out mice die at midgestation, and thus cannot be used to analyze the role of WT1 in podocyte maintenance. Therefore, a transgenic mouse model was generated, which allows for an inducible podocyte-specific knock-out of *WT1* in adult animals. For this an allele encoding a podocyte-specific doxycycline-inducible transcriptional activator (rtTA) was combined with an rtTA-inducible Cre allele, and a conditional *WT1* allele **(****Fig. 4a, b).** Knock-out efficiency was about 80% after two weeks of induction **(****Fig. 4c****).** The mice developed increasing albuminuria **(****Fig. 4d****)** and progressive flattening of foot processes and FSGS **(****Fig. 4e, f)** which is consistent with recently reported results for ubiquitous *WT1* knock-out in adults (Chau et al., supra). Deletion of WT1 was accompanied by loss of podocalyxin and nephrin detected by immunofluorescence **(****Fig. 4g, h).** Analyses of the targets of the WT1 knock-out revealed similar genes as also found in miR-193a overexpressing glomeruli **(Supplementary Table** 3) .

### Example 21: Re-expression of WT1 rescues miR-193a target genes.

Over-expression of miR-193a in cultured mouse podocytes reproduced the effects seen *in vivo* with down-regulation of WT1, podocalyxin, nephrin, and other WT1 target genes **(****Fig. 5a****).** By forced re-expression of WT1 most of these defects were rescued at both RNA **(****Fig. 5a****)** and protein levels **(****Fig. 5b****).** Forced re-expression of WT1 rescued most of these defects **(****Fig. 5a and 5b****).** These rescue experiments further confirm that WT1 exerts a crucial role in gene regulation in podocytes downstream of miR-193a.

### Example 22: Selective expression of miR-193a in human FSGS.

Over-expression of miR-193a in cultured human podocytes strongly reduced the expression levels of WT1 **(****Fig. 6a****),** similar to results obtained in mice. Inhibition of miR-193a by complementary LNA did not significantly enhance WT1 levels **(****Fig. 6a****).** Paraffin sections of kidney biopsies of 91 patients with FSGS and other primary glomerular diseases, including minimal change disease (MCD) were selected, as well as kidney tissue without glomerular pathology. Glomeruli were isolated by laser capture microdissection and miR-193a was quantified by qPCR. This analysis revealed that the level of miR-193a in glomeruli of FSGS patients exceeded that of all other groups with high statistical significance (p=0.00004) **(****Fig. 6b****).** There was no correlation between miR-193a expression and histological subtypes of FSGS1, but miR-193a was not overexpressed in cases with hereditary FSGS. By contrast, two of the four cases of HIV-related FSGS expressed high levels of miR-193a **(****Fig. 6c****).** In normal glomeruli **(****Fig. 6d****)** miR-193a was localized to the capsular epithelial cells and only very scarcely in podocytes **(****Fig. 6e****).** In FSGS patients with typical glomerular pathology **(****Fig. 6f****)** over-expression of miR-193a was confirmed in podocytes by fluorescence *in situ* hybridization **(****Fig. 6g****).** Expression levels of miR-193a in MCD were very low **(****Fig. 6e****).** In FSGS, loss of podocalyxin and WT1 was also observed by immunoflourescence **(****Fig. 6f, g),** similar to miR-193a over-expressing mice. Finally miR-193a as potential biomarker for FSGS in urine, but highly variable results were obtained without consistent correlation to diseases.

### Example 23: Summary

Inducible up-regulation of miR-193a in transgenic mice led to rapidly progressing glomerular disease and death from renal failure within 10 weeks. Mechanistically, miR-193a binds to the WT1 within its coding sequence and represses expression of WT1 - a protein essential for the development and maintenance of normal podocytes and glomeruli. In humans, genetic defects in WT1 cause the Denys-Drash and Frasier syndromes that are associated with effaced podocytes, similar to those found in FSGS. Although these results indicate that downregulation of WT1 by miR-193a is sufficient to induce FSGS, the possibility remains that other putative miR-193a target genes could contribute to the severity of miR-193a-induced podocyte damage. In this regard, RNA-Seq profiling revealed another target of miR-193a, the basic helix-loop-helix transcription factor *ATOH8* (atonal homolog 8, *Drosophila melanogaster*) that is associated with down-regulation of nephrin, synaptopodin, and glomerulosclerosis (Ross et al. Dev Dyn. 235, 3102-9 (2006)). Despite this, inducible knock-out of WT1 in mature mice recapitulated the miR-193a phenotype and the WT1-mediated rescue discounts essential contributions from other putative miR-193a targets.

Down-regulation of WT1 led to disruption of the podocyte structure due to the loss of WT1 target genes, including the podocyte membrane sialoglycoprotein podocalyxin, and the main slit diaphragm protein nephrin. Podocalyxin is a CD34-related sulphated sialoglycoprotein that is densely packed in the luminal/urinary podocyte cell membrane domain. It determines the overall electronegative glomerular charge (collectively designated as "glomerular polyanion") and is thought to keep the filtration slits between podocytes open by electrostatic repulsion. Its correct density and sialylation are critical for the control of podocyte shape, presumably by organizing the actin cytoskeleton to which it is linked via the scaffolding proteins HNERF and ezrin. Accordingly, genetic deletion of *podocalyxin* causes complete flattening of foot processes. The present results show that both podocalyxin mRNA and protein are lost upon miR-193a over-expression in cultured podocytes and isolated mouse glomeruli, indicating that loss of podocalyxin via WT1 suppression is one of the major effector mechanisms of miR-193a.

Nephrin is the main constituent of the podocyte's intercellular junctions, the slit diaphragms that serve as signalling platform for the control of podocyte stability and the gene that encodes it is a direct WT1 target and down-regulated upon loss of WT1. Mutations of nephrin were originally defined as the pathogenic cause of the congenital nephrotic syndrome, Finnish type. Genetic deletion of nephrin in mice results in a podocyte phenotype similar to that found in human FSGS. Thus, the down-regulation of *nephrin* by miR-193a via WT1 suppression is another powerful destabilizing event for podocytes.

In the present experiments miR-193a-dependent suppression of WT1 initiated a cascade leading to the widespread loss of structural podocyte proteins even though their genes lack binding sites for miR-193a or WT1. For example, down-regulation of several relevant transcripts were observed, such as synaptopodin and CD2AP. Loss of each of these proteins individually destabilizes the podocyte cytoskeleton and architecture. Global loss of podocyte stabilizing proteins has been documented in HIV-associated FSGS and ascribed to disruption of an undefined interdependent podocyte stabilizing mechanisms. The findings reported here, the central regulatory role of WT1 in the expression of these proteins is highlighted by the finding that their miR-193a-induced loss was rescued by forced over-expression of WT1.

In isolated glomeruli that were microdissected from biopsies of FSGS patients (n= 49) miR-193a was significantly enhanced in >70% of patients above the mean levels of all non-FSGS cases. Cases with hereditary background clustered in the range of controls, while two of four HIV-related cases showed high expression levels.

Previous reports as well as the current study have shown that in human FSGS podocalyxin and nephrin are reduced or lost, and this correlates with the present findings in podocytes of miR-193a over-expressing mice. Collectively, the present results provide a strong case that up-regulation of miR-193a is pathogenic in human FSGS and contributes to the podocyte damage.

In conclusion, the combined results obtained from *in vitro* experiments, mouse models and human tissues indicate that increased levels of miR-193a can initiate a cascade of podocyte-destabilizing molecular events, starting with the down-regulation of WT1, followed by the WT1 targets podocalyxin and nephrin, and eventually of other functionally important podocyte proteins. This cascade can be halted by inhibiting miR-193a. Several important podocyte target genes were affected by over-expression of miR-193a or knock-out of the WT1 gene. Mechanistically, binding of miR-193a to the coding region of WT1 acts as negative regulator, and subsequently causes lack of WT1 and hence reduces the transcription of podocalyxin and nephrin.

## Claims

1. Method of prophylactically treating a glomerular kidney disease or of reducing the risk of glomerular kidney disease in a patient comprising inhibiting miR-193a in a kidney cell in said patient.

2. MiR-193a inhibitor for use in treating a glomerular kidney disease or of reducing the risk of glomerular kidney disease in a patient comprising administering said inhibitor to said patient.

3. Method of claim 1 or miR-193a inhibitor for use according to claim 2, wherein said miR-193a inhibitor is a nucleic acid at least 90% complementary with a miR-193a sequence or a vector encoding said nucleic acid, wherein said vector causes expression of said nucleic acid inhibitor in a cell.

4. Method or miR-193a inhibitor for use according to claim 3, wherein said miR-193a sequence comprises at least 16 consecutives nucleotides of any mir-193a sequence, preferably a sequence as set forth in SEQ ID NO: 1, SEQ ID NO: 2, SEQ ID NO:3 or SEQ ID NO: 11.

5. Method or miR-193a inhibitor for use according to claim 3 or 4, wherein said nucleic acid inhibitor is 15 to 30 nucleotides in length.

6. Method of claim 1 or miR-193a inhibitor for use according to any one of claims 2 to 5, wherein said inhibitor is DNA or RNA.

7. Method of claim 1 or miR-193a inhibitor for use according to any one of claims 2 to 6, wherein said inhibitor comprises a nucleic acid modification which increases nuclease resistance or increases binding affinity with a miR-193a sequence as determined by a decreases the melting temperature of the hybridized complex of the miR-193a inhibitor with the miR-193a sequence as compared to a miR-193a inhibitor without said modification.

8. Method of claim 1 or miR-193a inhibitor for use according to any one of claims 2 to 7, wherein said glomerular kidney disease is further defined as glomerulosclerosis or glomerulonephritis, preferably further as glomerular kidney diseases associated with abnormal glomerular cell proliferation and/or scaring, focal segmental glomerulosclerosis, Frasier syndrome, Denys-Drash syndrome, congenital nephrotic syndrome, crescentic glomerulonephritis or mesangial sclerosis.

9. Method of claim 1 or miR-193a inhibitor for use according to any one of claims 2 to 8, wherein said kidney cell is a podocyte cell.

10. Method of reducing or eliminating miR-193a activity in a kidney cell comprising introducing a miR-193a inhibitor into said kidney cell.

11. The method of claim 10, wherein said method is an in vitro method.

12. The method of claim 10 or 11 further comprising comparing one or more characteristics of a kidney cell having the miR-193a inhibitor with a kidney cell not having the miR-193a inhibitor and/or assaying the cell for expression of mature miR-193a.

13. Method of diagnosing glomerular kidney disease or the risk of glomerular kidney disease comprising determining the expression level of miR-193a, wherein said glomerular kidney disease or risk thereof is detected if miR-193a expression levels are increased as compared to a healthy control.

14. The method of claim 13 wherein said miR-193a expression level is determined by analysing urine sediment, preferably cells.

15. A kit for performing a method according to claim 13 or 14, comprising a probe with at least 90% complementary with a miR-193a sequence, wherein said miR-193a sequence comprises at least 16 consecutives nucleotides of any miR-193a sequence, preferably a sequence as set forth in SEQ ID NO: 1, SEQ ID NO: 2, SEQ ID NO: 3 or SEQ ID NO: 11, a sample holding means, preferably a centrifugation vial, and one or more comparative sample holding means with a comparative miR-193a amount of a healthy control and/or of a kidney diseased reference, preferably wherein said disease is selected from the diseases as defined in claim 8.


SEQUENCE LISTING

<110>  IMBA - INSTITUT FÜR MOLEKULARE BIOTECHNOLOGIE GMBH

<120>  Therapeutic and diagnostic miRNA regulator in kidney disease

<130>  r62334

<160>  11

<170>  PatentIn version 3.3

<210>  1
<211>  88
<212>  RNA
<213>  homo sapiens

<400>  1
cgaggauggg agcugagggc ugggucuuug cgggcgagau gagggugucg gaucaacugg      60

ccuacaaagu cccaguucuc ggcccccg                                          88


<210>  2
<211>  22
<212>  RNA
<213>  homo sapiens

<400>  2
ugggucuuug cgggcgagau ga                                                22


<210>  3
<211>  22
<212>  RNA
<213>  homo sapiens

<400>  3
aacuggccua caaaguccca gu                                                22


<210>  4
<211>  20
<212>  DNA
<213>  Artificial

<220>
<223>  primer

<400>  4
cgatcaggat gatctggacg                                                   20


<210>  5
<211>  21
<212>  DNA
<213>  Artificial

<220>
<223>  primer

<400>  5
caagctcttc agcaatatca c                                                 21

<210>  6
<211>  25
<212>  DNA
<213>  Artificial

<220>
<223>  primer

<400>  6
ctcttccctc gtgatctgca actcc                                          25


<210>  7
<211>  24
<212>  DNA
<213>  Artificial

<220>
<223>  primer

<400>  7
catgtcttta atctacctcg atgg                                           24


<210>  8
<211>  28
<212>  DNA
<213>  Artificial

<220>
<223>  primer

<400>  8
ccgaccatct gaaaacgcac accaggac                                       28


<210>  9
<211>  28
<212>  DNA
<213>  Artificial

<220>
<223>  primer

<400>  9
gtcctggtgt gcgttttcag atggtcgg                                       28


<210>  10
<211>  8
<212>  DNA
<213>  homo sapiens

<400>  10
aagaccca                                                              8


<210>  11
<211>  22
<212>  RNA
<213>  Mus musculus

<400>  11
ugggucuuug cgggcaagau ga                                             22